# EUROPEAN PATENT SPECIFICATION

(11) **EP 4 181 693 B1**
(45) Date of publication and mention of the grant of the patent: **03.09.2025**
(21) Application number: 21746134.2
(22) Date of filing: 09.07.2021
(51) Int. Cl.: A23L 33/105, A23L 33/15, A61K 9/20, A61K 31/14, A61K 31/353, A61K 31/355, A61K 31/375, A61K 36/87, A61P 1/16, A61K 31/52, A61P 3/02, A61K 9/24, A61K 31/352

(54) **NUTRITIONAL SUPPLEMENT FOR USE IN A METHOD OF PROTECTION OF THE LIVER FROM THE EFFECTS OF THE USE OF METHOTREXATE AS A CHEMOTHERAPEUTIC DRUG, COMPRISING DIHYDROMYRICETIN, CHOLINE AND ONE OR MORE VITAMINS WITH ANTIOXIDANT ACTIVITY**
NAHRUNGSERGÄNZUNGSMITTEL ZUR VERWENDUNG IN EINEM VERFAHREN ZUM SCHUTZ DER LEBER VOR DEN WIRKUNGEN DER VERWENDUNG VON METHOTREXAT ALS CHEMOTHERAPEUTIKUM, DAS DIHYDROMYRICETIN, CHOLIN UND EIN ODER MEHRERE VITAMINE MIT ANTIOXIDATIVER WIRKUNG ENTHÄLT
SUPPLÉMENT NUTRITIONNEL À UTILISER DANS UNE MÉTHODE DE PROTECTION DU FOIE CONTRE LES EFFETS DE L'UTILISATION DU MÉTHOTREXATE COMME MÉDICAMENT CHIMIOTHÉRAPEUTIQUE, COMPRIS DE DIHYDROMYRICETIN, DE CHOLINE ET D'UNE OU PLUSIEURS VITAMINES AYANT UNE ACTIVITÉ ANTIOXYDANTE

(30) Priority: 14.07.2020 GR 20200100411
(43) Date of publication of application: 24.05.2023
(73) Proprietor: Uni-Pharma Kleon Tsetis Pharmaceutical Laboratories S.A., 14564 Kifissia, Attica (GR)
(72) Inventor: TSETI, Ioulia, 145 61 Kifissia Attica (GR)
(74) Representative: Sonnenhauser, Thomas Martin
(86) International application number: PCT/GR2021/000046
(87) International publication number: WO 2022/013581

(56) References cited:
- US-A1- 2005 176 811
- US-A1- 2016 158 305
- US-A1- 2017 290 768
- US-A1- 2019 350 888
- US-B2- 9 211 298
- ZHANG JINGYAO ET AL: "Recent Update on the Pharmacological Effects and Mechanisms of Dihydromyricetin", FRONTIERS IN PHARMACOLOGY, vol. 9, 25 October 2018 (2018-10-25), XP055860025, DOI: 10.3389/fphar.2018.01204
- FAN LANLAN ET AL: "Metabolomics of the Protective Effect of Ampelopsis grossedentata and Its Major Active Compound Dihydromyricetin on the Liver of High-Fat Diet Hamster", EVIDENCE-BASED COMPLEMENTARY AND ALTERNATIVE MEDICINE, vol. 2020, 28 January 2020 (2020-01-28), US, pages 1 - 15, XP055860027, ISSN: 1741-427X, Retrieved from the Internet <URL:https://downloads.hindawi.com/journals/ecam/2020/3472578.pdf> DOI: 10.1155/2020/3472578

## Description

### Description of the invention

Nutritional supplement suitable for oral administration, in the form of a double-layer tablet, for oral administration, for use in a method of protection of the liver from the effects of the use of methotrexate as a chemotherapeutic drug, said tablet comprising dihydromyricetin, choline and one or more vitamins with antioxidant activity as defined in the claims; wherein dihydromyricetin is optionally in the form of *Ampelopsis grossedentata* extract, choline, wherein choline is optionally in the form of choline bitartrate, and wherein the one or more vitamins with antioxidant activity are optionally selected from vitamin C, vitamin E or a combination of them. The nutritional supplement of the present invention is suitable for the maintenance of the normal liver function.

*Ampelopsis grossedentata* is a pharmaceutical plant growing mainly in southern China. Its leaves and stems are traditionally used as a health tea for the prevention and treatment of common cold symptoms, like sore throat and cough, as well as for pain in the mouth, pharynx and larynx. Dihydromyricetin is the main flavonoid found and isolated from the herb, *Ampelopsis grossedentata.* Recent studies demonstrated that dihydromyricetin possesses multiple health-benefit activities, including antioxidative, anti-inflammatory and antimicrobial effects, while is also active in lipid and glucose-metabolism-regulatory activities [Li, H. et al. The Versatile Effects of Dihydromyricetin in Health. Evidence-Based Complementary and Alternative Medicine, 2017, Article ID 1053617, 10 pages].

Choline is a quaternary ammonium salt (2-hydroxyethyl-N,N,N-trimethylammonium salt) which is either counterbalanced by an anion, i.e., a chloride to form choline chloride or is bound to an organic group, i.e., an acetyl- or a phosphatidyl-group, forming acetylcholine or phosphatidylcholine, respectively. Choline in its various forms occurs in foods (milk, eggs, peanuts) while it is synthesized *de novo* by the human body. According to the European Food Safety Authority (EFSA), choline contributes to normal homocysteine and lipid metabolism as well as to the maintenance of the normal liver function.

Vitamin C (L-ascorbic acid) is a water-soluble vitamin mainly found in citrus fruits and vegetables. Vitamin C deficiency is associated with hypovitaminosis and scurvy, which mainly causes vascular lesions, bleeding gums, tiredness and weakness. Vitamin C is a powerful antioxidant and protects the human body from free radicals activity. Furthermore, vitamin C participates in the hemoglobin production and improves the body's iron absorption from food. Although, Vitamin C is water soluble, however, it is unstable after long storage in aqueous solutions due to oxidation reactions, furthermore, it is quite sensitive to increased temperatures.

Vitamin E is a fat-soluble vitamin which is present in milk, vegetable oils and nuts, while its deficiency is related to muscle weakness. Vitamin E comprises a group of four tocopherols, consisting of a chromanol ring and an isoterpene side chain. The main biological activity of vitamin E is to neutralize free radicals in the body and to prevent the oxidation of other essential components such as polyunsaturated fatty acids and vitamin C.

The liver is the most important organ for the metabolism in the human body and it is responsible for amino acid synthesis and protein synthesis such as albumin and globulins. Among other vital functions, liver produces bile, which helps the absorption of food and its nutrients, as well as the storage of essential vitamins and minerals. Furthermore, liver removes toxic substances from the blood such as alcohol, medicines and ammonia. Gluconeogenesis, another important function of the liver, is defined as the synthesis of glucose from amino acids which occurs if blood sugar levels tend to decrease. Finally, the liver plays a key role in cholesterol homeostasis since it participates in lipid metabolism.

The most usual liver disease is viral hepatitis, which is characterized by inflammation and necrosis of the liver parenchyma leading to the destruction of liver cells. The most common causes of viral hepatitis are the five unrelated viruses hepatitis A, B, C, D, and E, which differ each other both in the mode of transmission and in clinical significance. Chronic hepatitis is the major risk factor for liver fibrosis, liver cirrhosis or hepatocellular carcinoma.

CN102775455A relates to the use of dihydromyricetin in the treatment of acute and chronic bronchitis.

CN104116730 relates to the use of dihydromyricetin in the treatment of Parkinson's disease.

WO2012027326A2 and US20190343776 relate to the use of dihydromyricetin in the treatment of alcohol intoxication and its side effects.

EP2872133 and WO2018/134713 relate to compositions comprising dihydromyricetin suitable for treating hair loss and inducing hair growth.

US2017/0290768 relates to nutritional supplements comprising more than thirty active ingredients, including dihydromyricetin and choline, which are suitably formulated to release them in the gastrointestinal tract.

US 2019/350888 A1 discloses a composition for use in the therapy of veisalgia, wherein the composition comprises at least one sugar compound, mineral salts, at least one compound for cell protection, at least one compound for promoting cell function, at least one compound for promoting detoxification, at least one neurotransmitter, at least one trace element, folic acid, optionally further trace elements and optionally a stimulating alkaloid.

US 9 211 298 B2 discloses compositions containing enriched and purified natural crocin and/or crocetin for prevention and/or treatment of cancers and other conditions and diseases, wherein the compositions comprise mainly enriched or purified natural crocin or crocetin or combination of both and possible other active phytochemicals.

US 2017/290768 A1 discloses a composition includes dietary supplements capable of reducing or reversing the negative effects of alcohol on motor and cognition, dietary supplements having anti-gastroparesis, antiemetic, analgesic and anti-inflammatory activities and/or dietary supplements capable of increasing alcohol catabolism and decreasing the level of toxic products of alcohol catabolism.

The article "Recent update on the Pharmacological Effects and Mechanisms of Dihydromyricetin" discloses pharmacological developments of dihydromyricetin.

The article "Metabolomics of the Protective Effect of Ampelopsis grossedentata and Its Major Active Compound Dihydromyricetin on the Liver of High-Fat Diet Hamster" discloses chemical data on the chemical compounds of *Ampelopsis grossedentata* and the effects of dihydromyricetin and *Ampelopsis grossedentata* on high-fat diet hamster livers.

US 2005/176811 A1 discloses the use of a vitamin combination for improving the tolerance to chemotherapy and/or radiotherapy of malignant tumors.

The combination of dihydromyristine, choline and one or more vitamins with antioxidant activity for use in the maintenance and enhancement of liver function, is not reported in the prior art.

The present invention relates to a nutritional supplement in the form of a double-layer tablet, suitable for oral administration, for use in a method of protection of the liver from the effects of the use of methotrexate as a chemotherapeutic drug, said tablet comprising dihydromyricetin, choline, optionally in the form of bitartrate, and one or more vitamins with antioxidant activity as defined in the claims. The nutritional supplement is suitable for the enhancement of normal liver function.

The nutritional supplement of the current invention comprising dihydromyricetin, choline and one or more vitamins with antioxidant activity significantly enhances the normal liver function and promotes the normal lipid metabolism providing strong antioxidant protection in liver cells.

The nutritional supplement of the present invention comprising of dihydromyricetin, choline and one or more vitamins with antioxidant activity provides enhanced action on the protection and the maintenance of normal liver function, compared to compositions which consisting of dihydromyricetin only or a combination of dihydromyricetin and choline.

Additionally, it was found that the combination of dihydromyricetin and choline in the presence of two vitamins with antioxidant activity at the same time, presents even better results in the maintenance of normal liver function, especially when the two vitamins with antioxidant activity are vitamin C and vitamin E.

The nutritional supplement of the current invention protects the liver from the effects of methotrexate.

The nutritional supplement of the current invention comprises a natural extract of *Ampelopsis grossedentata,* which is standardized to at least 95 % w/w dihydromyricetin by extract weight.

According to the present invention, the nutritional supplement comprising dihydromyricetin, choline and one or more vitamins with antioxidant activity, comprises an extract of *Ampelopsis grossedentata* in a percentage from 8.5 % to 16.0 % w/w of the total weight of the composition, preferably from 10.5 % to 14.0 % w/w of the total weight of the composition, more preferably is 12.5 % w/w of the total weight of the composition.

According to the present invention, the nutritional supplement comprising dihydromyricetin, choline and one or more vitamins with antioxidant activity, comprises dihydromyricetin in a percentage of from 8.0 % to 16.0 % w/w of the total weight of the composition, preferably from 10.0 % to 14.0 % w/w of the total weight of the composition, more preferably is 12.2 % w/w of the total weight of the composition.

In a preferred embodiment, the nutritional supplement of the present invention comprises an extract of *Ampelopsis grossedentata,* standardized in 98 % dihydromyricetin. In a preferred embodiment, the nutritional supplement of the present invention comprises 150 mg of an *Ampelopsis grossedentata* extract standardized in 98 % dihydromyricetin, which corresponds to 147 mg dihydromyricetin.

According to the present invention, the nutritional supplement comprising dihydromyricetin, choline and one or more vitamins with antioxidant activity, comprises choline in a percentage from 4.0 % to 10% w/w of the total weight of the composition, preferably in a percentage from 6.0 % to 8.0 % w/w of the total weight of the composition, more preferably is 6.9 % w/w of the total weight of the composition.

In a preferred embodiment, the nutritional supplement comprising dihydromyricetin, choline and one or more vitamins with antioxidant activity, comprises choline, in the form of choline bitartrate.

In a preferred embodiment, the nutritional supplement comprising dihydromyricetin, choline and one or more vitamins with antioxidant activity, comprises choline bitartrate in a percentage from 10.0 % to 24.0 % w/w of the total weight of the composition, preferably in a percentage from 15.0 % to 20.0 % w/w of the total weight of the composition, more preferably is 16.8 % w/w of the total weight of the composition.

According to the present invention, the nutritional supplement comprising dihydromyricetin, choline and one or more vitamins with antioxidant activity, comprises one or more vitamins selected from vitamin C and vitamin E.

In a preferred embodiment, the nutritional supplement comprising dihydromyricetin, choline and one or more vitamins with antioxidant activity, comprises vitamin C in a percentage from 6.0 % to 14.0 % w/w of the total weight of the composition, preferably in a percentage from 6.5 % to 10.0 % w/w of the total weight of the composition.

In a preferred embodiment, the nutritional supplement comprising dihydromyricetin, choline and one or more vitamins with antioxidant activity, comprises vitamin E in a percentage from 0.5 % to 5.0 % w/w of the total weight of the composition, preferably in a percentage from 0.8 % to 3.0 % w/w of the total weight of the composition.

In a preferred embodiment, the nutritional supplement comprising dihydromyricetin, choline and one or more vitamins with antioxidant activity, comprises both vitamin C and vitamin E.

In a preferred embodiment, the nutritional supplement comprising dihydromyricetin, choline and one or more vitamins with antioxidant activity, comprises 6.7 % w/w vitamin C and 1.0% w/w vitamin E, in relation to the total weight of the composition.

In a preferred embodiment, the nutritional supplement comprising dihydromyricetin, choline and one or more vitamins with antioxidant activity, comprises 12.2 % w/w dihydromyricetin, 6.9% w/w choline, 6.7% w/w vitamin C and 1.0% w/w vitamin E, in relation to the total weight of the composition.

According to the present invention, the nutritional supplement comprising dihydromyricetin, choline and one or more vitamins with antioxidant activity, is formulated in various solid forms including oral granules, tablets and hard capsules.

According to the present invention, the nutritional supplement comprising dihydromyricetin, choline and one or more vitamins with antioxidant activity, further comprises pharmaceutically acceptable excipients, selected from diluents, binders, lubricants and anticaking agents.

According to the present invention, the nutritional supplement comprising dihydromyricetin, choline and one or more vitamins with antioxidant activity, comprises a diluent selected from mannitol, maltitol and sorbitol in a percentage from 20 % to 30 % w/w of the total weight of the composition, preferably in a percentage from 22 % to 26 % w/w of the total weight of the composition.

According to the present invention, the nutritional supplement comprising dihydromyricetin, choline and one or more vitamins with antioxidant activity, comprises sorbitol as diluent in a percentage of 23 % w/w of the total weight of the composition.

According to the present invention, the nutritional supplement comprising dihydromyricetin, choline and one or more vitamins with antioxidant activity, comprises a binder selected from microcrystalline cellulose, pregelatinized starch, polyvinylpyrrolidone, anhydrous lactose or a combination of them, in a percentage from 25 % to 50 % w/w of the total weight of the composition, preferably in a percentage from 30 % to 40 % w/w of the total weight of the composition.

In a preferred embodiment, the nutritional supplement comprising dihydromyricetin, choline and one or more vitamins with antioxidant activity, comprises microcrystalline cellulose in a percentage of 38 % w/w of the total weight of the composition, as a binder.

In a preferred embodiment, the nutritional supplement comprising dihydromyricetin, choline and one or more vitamins with antioxidant activity, comprises as binders both microcrystalline cellulose in a percentage of 33.5 % w/w and pregelatinized starch in a percentage of 4.5 % w/w of the total weight of the composition.

According to the present invention, the nutritional supplement comprising dihydromyricetin, choline and one or more vitamins with antioxidant activity comprises a lubricant, selected from polyethylene glycol 4000, polyethylene glycol 6000 and magnesium stearate, in a percentage from 1.0% to 3.5% w/w of the total weight of the composition.

In a preferred embodiment, the nutritional supplement comprising dihydromyricetin, choline and one or more vitamins with antioxidant activity, comprises magnesium stearate in a percentage of 1.25 % w/w of the total weight of the composition, as a lubricant.

According to the present invention, the nutritional supplement comprising dihydromyricetin, choline and one or more vitamins with antioxidant activity comprises an anticaking agent selected from magnesium carbonate, silicon dioxide, magnesium sulfate and magnesium oxide, in a percentage from 0.5 % to 3 % w/w of the total weight of the composition.

In a preferred embodiment, the nutritional supplement comprising dihydromyricetin, choline and one or more vitamins with antioxidant activity, comprises silicon dioxide in a percentage of 0.75 % w/w of the total weight of the composition, as anticaking agent.

The nutritional supplement of the present invention is further described with the following representative but non-limiting example.

**Example 1.** Nutritional supplement comprising dihydromyricetin, choline bitartrate, vitamin C and vitamin E in the form of a double-layer tablet.

*First layer composition.* Choline L-bitartrate (1.2 Kg) is mixed with sorbitol (0.6 Kg) for 15 min. Microcrystalline cellulose (1.32 Kg), pregelatinized starch (0.13 Kg) and silicon dioxide (0.02 Kg) are sequentially added and the new blend is mixed for 15 minutes. Magnesium stearate (0.03 Kg) is then added to the final blend and mixing is continued for another 5 minutes.

*Second layer composition. Ampelopsis grossedentata* extract (0.90 Kg), vitamin C (0.48 Kg) and vitamin E (0.072 Kg) are mixed in the presence of sorbitol (1.05 Kg) for 15 minutes. Microcrystalline cellulose (1.1 Kg), pregelatinized starch (0.20 Kg) and silicon dioxide (0.035 Kg) are sequentially added and the new blend is further stirred for 15 minutes. Then, magnesium stearate (0.06 Kg) is added and the final blend stirred further for 5 minutes.

Finally, a tablet press machine with the ability to produce multilayer tablets is used to produce 6000 double-layer tablets according to the following specifications:

| | |
|---|---|
| Average tablet weight: | 1200 gr |
| Average weight of the first layer: | 550 mg |
| Average weight of the second layer: | 650 mg |

According to the present embodiment, a bilayer tablet of the nutritional supplement comprising dihydromyrisetin, choline bitartrate, vitamin C and vitamin E has the following composition:

| **Nr** | **Ingredients** | **Function** | **Quantity (mg)** |
|---|---|---|---|
| 1 | *Ampelopsis grossedentata* extract | Active substance | 150.00* |
| 2 | Choline Bitartrate | Active substance | 201.00** |
| 3 | Vitamin C | Active substance | 80.00 |
| 4 | Vitamin E | Active substance | 12.00 |
| 6 | Sorbitol solid | Diluent | 275.00 |
| 7 | Microcrystalline cellulose | Binder | 403.00 |
| 8 | Starch pregelatinized | Binder | 55.00 |
| 9 | Magnesium stearate | Lubricant | 15.00 |
| 10 | Silicon dioxide | Anticaking agent | 9.00 |

| | | | |
|---|---|---|---|
| * The amount corresponds to 147 mg dihydromyricetin, ** The amount corresponds to 82.5 mg choline | | | |

Each bilayer tablet comprises an amount of active substance corresponding to one dosage unit.

Each bilayer tablet provides 100% of the recommended dietary allowance for vitamins C and vitamin E.

There is strong evidence that the daily consumption of two tablets described in Example 1, significantly enchases normal liver function and promotes the normal lipid metabolism, while offering strong antioxidant protection to liver cells.

In addition, it is found that the more defined are the limits of the amount of vitamin C and vitamin E used in the nutritional supplement comprising dihydromyricetin, choline, vitamin C and vitamin E, the more efficient is in the antioxidant protection of liver cells.

## Claims

1. Nutritional supplement, in the form of a double-layer tablet for oral administration, for use in a method of protection of the liver from the effects of the use of methotrexate as a chemotherapeutic drug, said tablet comprising dihydromyricetin, choline and one or more vitamins with antioxidant activity.

2. The nutritional supplement for use according to claim 1, wherein the vitamins with antioxidant activity are selected from vitamin C and vitamin E or a combination of them.

3. The nutritional supplement for use according to any of claims 1 and 2, comprising dihydromyricetin in a percentage from 8.0 % to 16.0 % w/w, preferably from 10.0 % to 14.0 % w/w of the total weight of the composition.

4. The nutritional supplement for use according to any of claims 1 to 3, comprising choline in a percentage from 4.0 % to 10% w/w, preferably from 6.0 % to 8.0 % w/w of the total weight of the composition.

5. The nutritional supplement for use according to any of claims 1 to 4, comprising vitamin C in a percentage from 6.0 % to 14.0 % w/w, preferably from 6.5 % to 10.0 % w/w of the total weight of the composition.

6. The nutritional supplement for use according to any of claims 1 to 5, comprising vitamin E in a percentage from 0.5 % to 5.0 % w/w, preferably from 0.8 % to 3.0 % w/w of the total weight of the composition.

7. The nutritional supplement for use according to any of claims 1 to 6, comprising dihydromyricetin in a percentage of 12.2 % w/w, choline in a percentage of 6.9% w/w, vitamin C in a percentage of 6.7% w/w and vitamin E in a percentage of 1.0% w/w of the total weight of the composition.

8. The nutritional supplement for use according to any of claims 1 to 7, further comprising pharmaceutically acceptable excipients selected from diluents, binders, lubricants and anticaking agents.

9. The nutritional supplement for use according to any of claims 1 to 8, wherein one dosage unit comprises 147 mg dihydromyricetin, 82.5 mg choline, 80 mg vitamin C and 12 mg vitamin E.

## Patentansprüche

1. Nahrungsergänzungsmittel in Form einer doppelschichtigen Tablette zur oralen Verabreichung zur Verwendung in einem Verfahren zum Schutz der Leber vor den Wirkungen der Verwendung von Methotrexat als chemotherapeutisches Medikament, wobei die Tablette Dihydromyricetin, Cholin und ein oder mehrere Vitamine mit antioxidativer Aktivität enthält.

2. Nahrungsergänzungsmittel zur Verwendung nach Anspruch 1, wobei die Vitamine mit antioxidativer Wirkung ausgewählt sind aus Vitamin C und Vitamin E oder einer Kombination davon.

3. Nahrungsergänzungsmittel zur Verwendung nach einem der Ansprüche 1 und 2, umfassend Dihydromyricetin in einem Prozentsatz von 8,0 % bis 16,0 % (w/w), vorzugsweise von 10,0 % bis 14,0 % (w/w) des Gesamtgewichts der Zusammensetzung.

4. Nahrungsergänzungsmittel zur Verwendung nach einem der Ansprüche 1 bis 3, umfassend Cholin in einem Prozentsatz von 4,0 % bis 10 % w/w, vorzugsweise von 6,0 % bis 8,0 % w/w des Gesamtgewichts der Zusammensetzung.

5. Nahrungsergänzungsmittel zur Verwendung nach einem der Ansprüche 1 bis 4, umfassend Vitamin C in einem Prozentsatz von 6,0 % bis 14,0 % w/w, vorzugsweise von 6,5 % bis 10,0 % w/w des Gesamtgewichts der Zusammensetzung.

6. Nahrungsergänzungsmittel zur Verwendung nach einem der Ansprüche 1 bis 5, umfassend Vitamin E in einem Prozentsatz von 0,5 % bis 5,0 % w/w, vorzugsweise von 0,8 % bis 3,0 % w/w, bezogen auf das Gesamtgewicht der Zusammensetzung.

7. Nahrungsergänzungsmittel zur Verwendung nach einem der Ansprüche 1 bis 6, umfassend Dihydromyricetin in einem Anteil von 12,2 % w/w, Cholin in einem Anteil von 6,9 % w/w, Vitamin C in einem Anteil von 6,7 % w/w und Vitamin E in einem Anteil von 1,0 % w/w des Gesamtgewichts der Zusammensetzung.

8. Nahrungsergänzungsmittel zur Verwendung nach einem der Ansprüche 1 bis 7, ferner umfassend pharmazeutisch annehmbare Hilfsstoffe, ausgewählt aus Verdünnungsmitteln, Bindemitteln, Gleitmitteln und Antibackmitteln.

9. Nahrungsergänzungsmittel zur Verwendung nach einem der Ansprüche 1 bis 8, wobei eine Dosierungseinheit 147 mg Dihydromyricetin, 82,5 mg Cholin, 80 mg Vitamin C und 12 mg Vitamin E umfasst.

## Revendications

1. Complément nutritionnel, sous forme de comprimé bicouche destiné à l'administration orale, pour une utilisation dans une méthode de protection du foie contre les effets de l'utilisation du méthotrexate comme médicament chimiothérapeutique, ledit comprimé comprenant de la dihydromyricétine, de la choline et une ou plusieurs vitamines à activité antioxydante.

2. Complément nutritionnel pour une utilisation selon la revendication 1, dans lequel les vitamines à activité antioxydante sont choisies parmi la vitamine C et la vitamine E, ou une combinaison de celles-ci.

3. Complément nutritionnel pour une utilisation selon l'une quelconque des revendications 1 et 2, comprenant de la dihydromyricétine en un pourcentage de 8,0 % à 16,0 % p/p, de préférence de 10,0 % à 14,0 % p/p du poids total de la composition.

4. Complément nutritionnel pour une utilisation selon l'une quelconque des revendications 1 à 3, comprenant de la choline en un pourcentage de 4,0 % à 10 % p/p, de préférence de 6,0 % à 8,0 % p/p, du poids total de la composition.

5. Complément nutritionnel pour une utilisation selon l'une quelconque des revendications 1 à 4, comprenant de la vitamine C en un pourcentage de 6,0 % à 14,0 % p/p, de préférence de 6,5 % à 10,0 % p/p, du poids total de la composition.

6. Complément nutritionnel pour une utilisation selon l'une quelconque des revendications 1 à 5, comprenant de la vitamine E en un pourcentage de 0,5 % à 5,0 % p/p, de préférence de 0,8 % à 3,0 % p/p, du poids total de la composition.

7. Complément nutritionnel pour une utilisation selon l'une quelconque des revendications 1 à 6, comprenant de la dihydromyricétine en un pourcentage de 12,2 % p/p, de la choline en un pourcentage de 6,9 % p/p, de la vitamine C en un pourcentage de 6,7 % p/p et de la vitamine E en un pourcentage de 1,0 % p/p du poids total de la composition.

8. Complément nutritionnel pour une utilisation selon l'une quelconque des revendications 1 à 7, comprenant en outre des excipients pharmaceutiquement acceptables choisis parmi les diluants, les liants, les lubrifiants et les agents antiagglomérants.

9. Complément nutritionnel pour une utilisation selon l'une quelconque des revendications 1 à 8, dans lequel une unité posologique comprend 147 mg de dihydromyricétine, 82,5 mg de choline, 80 mg de vitamine C et 12 mg de vitamine E.
